# EUROPEAN PATENT APPLICATION

(11) **EP 2 724 663 A1**
(43) Date of publication of application: **30.04.2014**
(21) Application number: 13189900.7
(22) Date of filing: 23.10.2013
(51) Int. Cl.: A61B 3/14

(54) **Ophthalmologic apparatus and control method**

(30) Priority: 26.10.2012 JP 2012236800
(71) Applicant: CANON KABUSHIKI KAISHA, Ohta-ku Tokyo 146-8501 (JP)
(72) Inventor: Kishida, Nobuyoshi, Ohta-ku, Tokyo (JP); Fujimori, Toshiya, Ohta-ku, Tokyo (JP)
(74) Representative: Derham, Cassandra Virginie

(57) **Abstract**

An ophthalmologic apparatus includes a focusing unit configured to focus, on an imaging unit, a return beam from a fundus of a subject's eye, a first drive unit configured to drive the focusing unit based on an index image obtained by imaging, using the imaging unit, a return beam from the fundus resulting from an index projected on the fundus by a projection unit, and a second drive unit configured to drive, after the first drive unit has driven the focusing unit, the focusing unit based on a contrast of a fundus image obtained by imaging, using the imaging unit, a return beam from the fundus, the fundus having been illuminated by an illumination unit.

## Description

### BACKGROUND OF THE INVENTION

### Field of the Invention

The present invention relates to an ophthalmologic apparatus and a control method.

### Description of the Related Art

In a conventional fundus camera, divided focus indices are projected on the fundus of the subject's eye to adjust a focus of an observation optical system on the fundus. Images of the focus indices are then observed via a focus lens in an observation imaging system, and the focus is adjusted by observing a positional relation between the divided focus index images. Further, the projected focus index images can be captured and auto-focusing can be performed based on the positional relation between the captured focus index images.

However, if the focus is adjusted by only setting the divided focus index images to a predetermined positional relation (e.g., align the divided focus images in a line), an error may occur. More specifically, an aberration in an optical system of the subject' s eye due to astigmatism may cause an error in focus adjustment, and a defocused fundus image may be obtained. To solve such a problem, Japanese Patent Application Laid-Open No. 2009-268772 discusses an ophthalmologic imaging apparatus as follows. The ophthalmologic imaging apparatus, after setting the focus index images to be of the predetermined positional relation, performs passive auto-focusing using contrast of the focus index images. The ophthalmologic imaging apparatus thus performs highly accurate auto-focusing.

However, the ophthalmologic imaging apparatus discussed in Japanese Patent Application Laid-Open No. 2009-268772 performs auto-focusing based on the focus indices. As a result, the effect of the aberration in the optical system of the subject's eye due to astigmatism may remain in regions of the fundus on which the focus indices are not projected.

On the other hand, Japanese Patent Application Laid-Open No. 2011-50532 discusses an ophthalmologic imaging apparatus which performs passive auto-focusing as follows. The ophthalmologic imaging apparatus performs line-of-sight detection and right and left eye detection, and predicts a position of a specific region (e.g., medium and large blood vessels (a choroidal stroma) on the fundus. The apparatus thus performs passive auto-focus using the contrast of the predicted specific region.

The ophthalmologic imaging apparatus discussed in Japanese Patent Application Laid-Open No. 2011-50532 performs auto-focusing using contrast with respect to the specific region on the fundus on which the focus index images are not projected. It can thus reduce, with respect to the specific region on the fundus, the effect of the aberration in the optical system of the subject's eye due to astigmatism.

However, according to Japanese Patent Application Laid-Open No. 2011-50532, the following becomes necessary in performing auto-focusing using the contrast. It becomes necessary to drive the focus lens over an entire focusing range of the fundus image of the subject's eye, and detect the contrast of the specific region on the fundus. As a result, time is required for detecting a focus position.

### SUMMARY OF THE INVENTION

The present invention is directed to an ophthalmologic apparatus capable of reducing the effect of aberration in the eye and the time required for detecting the focus position.

According to a first aspect of the present invention, there is provided an ophthalmologic apparatus as specified in claims 1 to 10. According to a second aspect of the present invention, there is provided a control method as specified in claim 11. According to a third aspect of the present invention, there is provided a program as specified in claim 12.

Further features of the present invention will become apparent from the following description of exemplary embodiments with reference to the attached drawings.

### BRIEF DESCRIPTION OF THE DRAWINGS

Fig. 1 illustrates an example of a configuration of an ophthalmologic imaging apparatus according to a first exemplary embodiment of the present invention.

Fig. 2 illustrates an example of a functional configuration according to the first exemplary embodiment.

Fig. 3 illustrates an example of a fundus image displayed on a monitor.

Fig. 4 illustrates an example of a relation between focus index images and contrast values in an area A301.

Fig. 5 illustrates an example of focus position detection performed by a first focus detection unit according to the first exemplary embodiment.

Fig. 6 illustrates a principle of contrast detection.

Fig. 7 is a flowchart illustrating an example of a control method according to the first exemplary embodiment.

Fig. 8 illustrates an example of the functional configuration according to a second exemplary embodiment of the present invention.

Fig. 9 illustrates an example of focus position detection performed by a second focus detection unit according to the second exemplary embodiment.

Fig. 10 is a flowchart illustrating an example of a control method according to the second exemplary embodiment.

### DESCRIPTION OF THE EMBODIMENTS

Various exemplary embodiments, features, and aspects of the invention will be described in detail below with reference to the drawings.

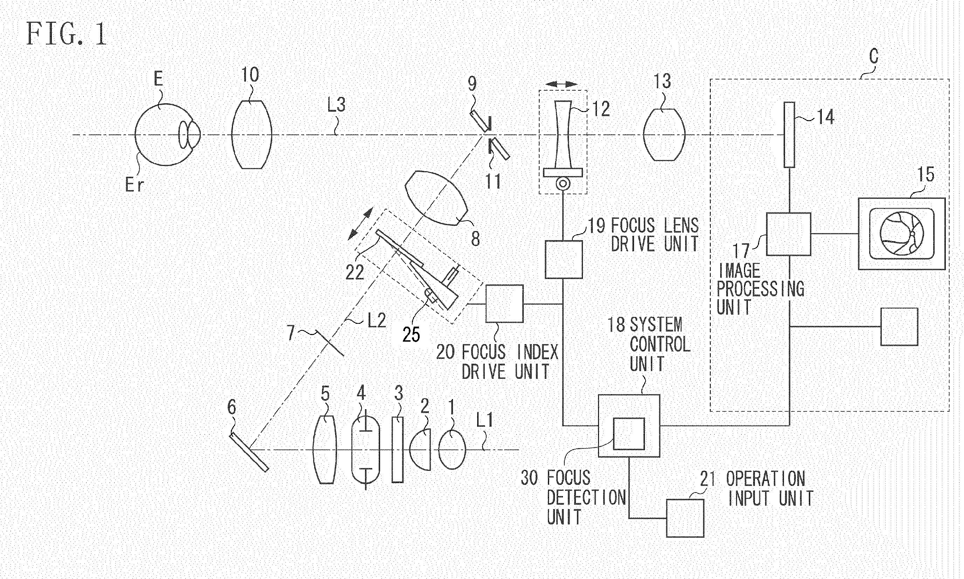

Fig. 1 illustrates an example of the configuration of the ophthalmologic apparatus (e.g., the fundus camera) according to the first exemplary embodiment.

Referring to Fig. 1, an observation light source 1, a condenser lens 2, a filter 3, an imaging light source 4, a lens 5, and a mirror 6 are arranged on an optical axis L1. The observation light source 1, such as a halogen lamp, emits fixed light. The filter 3 transmits infrared light and blocks visible light. The imaging light source 4 may be a flash unit. Further, a ring diaphragm 7, a focus index projection unit 22, a relay lens 8, and a perforated mirror 9 are sequentially arranged on an optical axis L2, which is in a reflecting direction of the mirror 6. The ring diaphragm 7 has a ring-shaped aperture. The perforated mirror 9 has a center aperture.

An objective lens 10 is arranged facing a subject's eye E on an optical axis L3, which is in the reflecting direction of the perforated mirror 9. A photographic diaphragm 11 is arranged in a perforated portion of the perforated mirror 9. A focus lens 12 and a photographic lens 13 are sequentially arranged on the optical axis L3. The focus lens 12 adjusts the focus by moving on the optical axis L3. The focus lens 12 corresponds to an example of a focusing unit configured to focus on an imaging unit a return beam from the fundus of the subject' s eye.

An image sensor 14, which performs moving image observation and still image capturing inside an imaging camera C, is disposed in an opposite direction to the subject's eye E with respect to the photographic lens 13. An output of the imaging sensor 14 is connected to an image-processing unit 17, and an output of the image-processing unit 17 is connected to a system control unit 18. The image-processing unit 17 thus displays on a monitor 15 an observation image captured by the image sensor 14. The image sensor 14 preferably performs both moving image observation and still image capturing. However, the present invention is not limited thereto. For example, an image sensor for performing moving image observation and an image sensor for performing still image observation may be separately disposed.

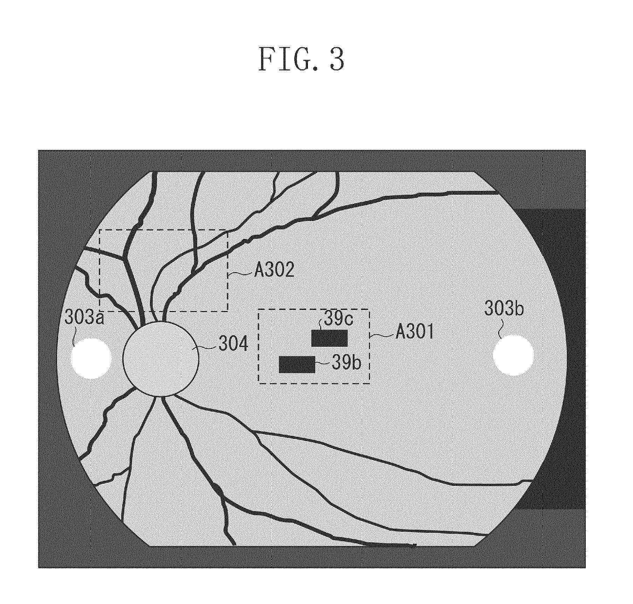

The focus index projection unit 22 is arranged between the ring diaphragm 7 and the relay lens 8 on the optical axis L2. The focus index projection unit 22 divides the light emitted from the light source (not illustrated) using a prism, and projects the divided light onto the subject's eye E. The divided light corresponds to an example of a first index and a second index. The image sensor 14 then captures the return beam from the subject's eye E, so that focus index images 39b and 39c illustrated in Fig. 3 are obtained. In other words, the focus index projection unit 22 corresponds to an example of a projection unit which projects an index onto the fundus. The focus index images 39b and 39c correspond to examples of the index images as obtained by the imaging unit. The imaging unit images the return beam from the fundus including the indices projected by the focus index projection unit. Furthermore, the focus index images 39b and 39c may hereinafter be respectively referred to as a first index image and a second index image.

A focus lens drive unit 19 and a focus index drive unit 20 respectively move the focus lens 12 and the focus index projection unit 22 in conjunction with each other in the directions of the optical axis L2 and the optical axis L3. The focus index projection unit 22 and the focus lens 12 are moved based on control of the system control unit 18. For example, the system control unit 18 moves the focus index projection unit 22 and the focus lens 12 in conjunction with each other so that a conjugate relation between the focus index projection unit 22 and the image sensor 14 is maintained.

When the ophthalmologic apparatus is in a manual focusing mode, the system control unit 18 controls the focus lens drive unit 19 and the focus index drive unit 20 according to an operation input using an operation input unit 21. In such a case, the focus index projection unit 22 and the image sensor 14 are optically conjugate. On the other hand, when the ophthalmologic apparatus is in an auto-focusing mode, the system control unit 18 controls the focus lens drive unit 19 and the focus index drive unit 20 based on a detection result of a focus detection unit 30 in the system control unit 18. In other words, in an auto-focusing case, the system control unit 18 drives the focus lens 12 and the focus index projection unit 22.

The system control unit 18 controls light intensity adjustment and on/off of the observation light source 1 and the imaging light source 4. Moreover, the system control unit 18 also controls the light intensity adjustment and on/off of a focus index illumination light-emitting diode (LED) 25. The system control unit 18 also controls, via the focus index drive unit 20, driving of the focus index projection unit 22. Furthermore, the system control unit 18 controls driving of the focus lens 12.

An example of the operation of the ophthalmologic apparatus according to the present exemplary embodiment will be described below.

The system control unit 18 turns on the observation light source 1. The condenser lens 2 condenses a light flux emitted from the observation light source 1, and the filter 3 filters out the visible light and transmits only the infrared light. The light transmitted through the filter 3 is then transmitted through the imaging light source 4, such as a flash unit, and becomes a ring-shaped flux via the lens 5, the mirror 6, and the ring diaphragm 7. The relay lens 8 and the perforated mirror 9 deflect the ring-shaped light flux in the direction of the optical axis L3. The ring-shaped light flux thus illuminates a fundus Er of the subject's eye E via the objective lens 10. The light flux reaching the fundus Er is reflected and scattered, and exits the subject's eye Er. The light emitted from the subject's eye E is formed into an image on the image sensor 14 via the objective lens 10, the photographic diaphragm 11, the focus lens 12, and the photographic lens 13. The image-processing unit 17 then displays on the monitor 14 the fundus image captured by the image sensor 14.

An operator observes the fundus image displayed on the monitor 15 and performs fine adjustment for aligning the subject's eye E and an optical unit including the members illustrated in Fig. 1 (excluding the subject's eye E, of course). The operator then performs focus adjustment, presses an imaging switch (not illustrated), and captures an image of the subject' s eye E. According to the present exemplary embodiment, the apparatus including an auto-focusing function which automatically executes the above-described focus adjustment will be described below.

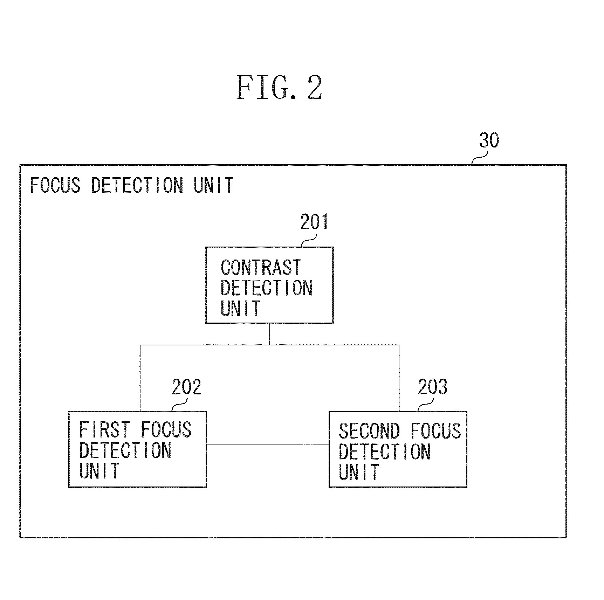

An example of the function of the focus detection unit 30 according to the first exemplary embodiment will be described below with reference to Fig. 2.

Referring to Fig. 2, the focus detection unit 30 detects the focus position of the focus lens. The focus detection unit 30 includes a contrast detection unit 201, a first focus detection unit 202, and a second focus detection unit 203 to be used in focusing. The contrast detection unit 201 is connected to the image sensor 14 of Fig. 1, the first focus detection unit 202 and the second focus detection unit 203. However, it is not necessary for the contrast detection unit 201 to be connected to the image sensor 14. For example, the contrast detection unit 201 may be connected to the image processing unit 17 instead. In such a case, the contrast detection unit 201 obtains the output from the image sensor 14 via the image processing unit 17.

The first focus detection unit 202 and the second focus detection unit 203 are connected to each other for synchronously starting focus detection. The first focus detection unit 202 and the second focus detection unit 203 thus both perform focus detection using the contrast detection unit 201.

The contrast detection unit 201 detects (i.e., calculates) the contrast of the fundus image output from the image sensor 14. More specifically, the contrast detection unit 201 calculates the contrast of a predetermined area on the fundus image detected by the first focus detection unit 202 and the contrast of a predetermined area on the fundus image detected by the second focus detection unit 203.

The first focus detection unit 202 detects the focus position using the positional relation between the focus index images 39b and 39c (refer to Fig. 3) to be described below. More specifically, the first focus detection unit 202 determines the area of the fundus image of which the contrast detection unit 201 is to calculate the contrast. For example, the first focus detection unit 202 determines the area including the focus index images 39b and 39c, to be described below, as the area where the contrast detection unit 201 is to calculate the contrast. If it is known that the focus index images 39b and 39c exist near the center of the fundus image, the first focus detection unit 202 determines the center area of the fundus image as the area where the contrast detection unit 201 is to calculate the contrast.

The system control unit 18 drives (i.e., moves) the focus index projection unit 22 and the focus lens 12 to the focus position detected by the contrast detection unit 201 in cooperation with the first focus detection unit 202. In other words, the system control unit 18, the contrast detection unit 201, and the first focus detection unit 202 function as an example of the first drive unit. More specifically, the first drive unit drives the focusing unit based on the index image obtained by imaging, using the imaging unit, the return beam from the fundus of the subject's eye of the indices projected on the fundus by the projection unit, which projects the indices on the fundus.

The second focus detection unit 203 detects the focus position using the contrast of the image of a fundus tissue. More specifically, the second focus detection unit 203 determines the area on the fundus image where the contrast detection unit 201 is to calculate the contrast. For example, the second focus detection unit 203 determines, as the area to be used in performing auto-focusing, the area in the fundus image which does not include the focus index images 39b and 39c. Further, the second focus detection unit 203 determines, as the area to be used in performing auto-focusing, the area including the medium and large blood vessels. The positions of the medium and large blood vessels can be estimated based on a macula lutea and an optic disk. As a result, the second focus detection unit 203 is capable of determining, as the area where the contrast detection unit 201 is to calculate the contrast, the area in the fundus image which does not include the focus index images 39b and 39c. The second focus detection unit 203 is capable of determining the area based on a projected position of a fixation target on the subject's eye.

The system control unit 18 drives (moves) the focus lens 12 to the focus position detected by the contrast detection unit 201 in cooperation with the second focus detection unit 203. In other words, the system control unit 18, the contrast detection unit 201, and the second focus detection unit 203 function as an example of the second drive unit. More specifically, the second drive unit drives, after the first drive unit has driven the focusing unit, the focusing unit based on the contrast of the fundus image obtained by imaging, using the imaging unit, a return beam from the fundus, the latter having been illuminated by the illumination unit. The system control unit 18 thus drives the focusing unit based on the contrast of a characteristic region (e.g., the fundus tissue such as the medium and large blood vessels) included in the fundus image.

Focus detection positions and ranges to be used by the first focus detection unit 202 and the second focus detection unit 203 will be described below with reference to Fig. 3. Fig. 3 is an enlarged view illustrating the fundus image displayed on the monitor 15. The area A301 is the focus detection position and range of the first focus detection unit 202. An area A302 is the focus detection position and range of the second focus detection unit 203.

The area A301 includes the focus index images 39b and 39c. According to the first exemplary embodiment, the fundus portion A302 to be detected by the second focus detection unit 203 is the area containing the medium and large blood vessels in the retina. However, it is not limited thereto, as long as the focus index images 39b and 39c on which the first focus detection unit 202 is to perform focus detection are not displayed in the same area. For example, the fundus portion to be detected by the second focus detection unit 203 may be an optic disc 304.

Further, the focus detection position and range used by the first focus detection unit 202 are not limited to the area A301, and other position and range in the fundus image may be used as long as the focus index images 39b and 39c are included therein.

An area 303a and an area 303b illustrated in Fig. 3 are alignment index images used for aligning the fundus image and the subject's eye. Since the alignment index images are not directly related to the auto-focusing function according to the present exemplary embodiment, detailed description will be omitted. It is desirable to determine the area A302 so that the alignment index images 303a and 303b are not included.

As described above with reference to Figs. 2 and 3, according to the present exemplary embodiment, the first focus detection unit 202 performs focus detection based on contrast detection of the focus index images. The second focus detection unit 203 then performs focus detection based on contrast detection of the fundus image in an area not containing the focus index images.

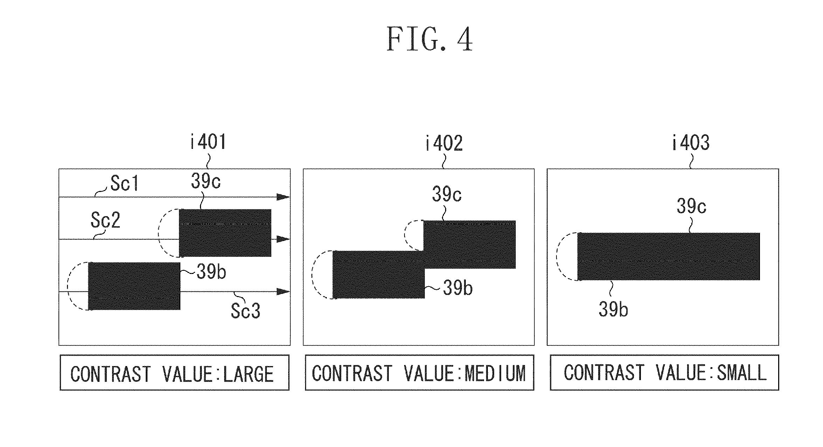

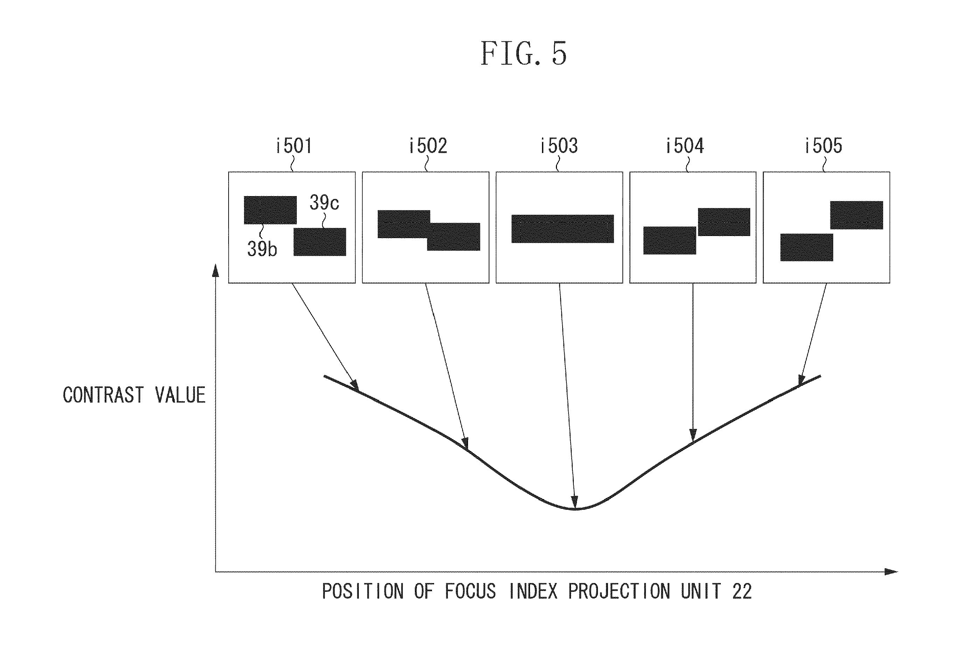

The first focus detection unit 202 will be described in detail below with reference to Figs. 4 and 5.

As described above, the area A301 illustrated in Fig. 3 is the focus detection area of the first focus detection unit 202. Referring to Fig. 4, images i401, i402, and i403 indicate the respective positions of the focus index images 39b and 39c in the area A301 illustrated in Fig. 3 obtained over time whilst the focus index projection unit 22 is driven.

Scan lines Sc1 and Sc2 in the image i401 indicate scanning performed by the contrast detection unit 201 for evaluating the contrast of the image. According to the present exemplary embodiment, the contrast is a luminance difference between adj acent pixels. Further, a contrast value is the value of a largest luminance difference among luminance data of the scan lines.

The arrows of the scan lines Sc1 and Sc2 indicate a scanning direction. The contrast detection unit 201 scans the lines horizontally and scans a number of horizontal lines corresponding to the number of pixels in a vertical direction from the top to the bottom of the image i401. The contrast detection unit 201 scans the lines from the left to the right in Fig. 4. However, it is not limited thereto, and the contrast detection unit 201 may scan from the right to the left. Further, the contrast detection unit 201 scans the lines according to the number of pixels from the top to the bottom in the vertical direction of the image i401. However, it is not limited thereto, and the contrast detection unit 201 may thin out the lines to be scanned.

The operation of the contrast detection unit 201 will be described below. For ease of description, it is assumed in the images i401, i402, and i403 that the luminance values of the focus index images 39b and 39c are the same, and the luminance values of the portions other than the focus index images 39b and 39c are the same. Further, only three scan lines Sc1, Sc2, and Sc3 are illustrated in Fig. 4 for ease of description. However, it is not limited thereto.

Since the focus index images 39b and 39c are not included in the scan line Sc1 in the image i401, the luminance values of all portions in the scan line Sc1 become the same. As a result, the contrast detection unit 201 calculates the contrast value in the scan line Sc1 as 0. The scan line Sc2 includes the focus index image 39c. The contrast detection unit 201 thus calculates, as the contrast value of the scan line Sc2, the difference (circled with a dotted line) between the luminance value of the portions other than the focus index images 39b and 39c and the luminance value of a left lateral side of the focus index image 39c.

For example, it is assumed that the luminance value of the portions other than the focus index images 39b and 39c is 0, and the luminance value of the focus index image 39c is 100. In such a case, the contrast value in the scan line Sc2 becomes 100. Further, the contrast detection unit 201 calculates as the contrast value of the scan line Sc3, the luminance difference of the left lateral side of the focus index image 39b. The contrast value thus becomes 100, similarly to the case of scan line Sc2.

The contrast value of the entire image i401 is then calculated as follows. The lines are scanned according to the number of the pixels from the top to the bottom of the image i401 in the vertical direction. A sum of the contrast values obtained for each line then becomes the contrast value of the entire image i401. For example, if a vertical length of one focus index image 39b corresponds to 10 scan lines, the contrast value becomes 100 × 10 = 1000. The contrast detection unit 201 thus obtains the contrast value of the entire image as described above. As a result, the portions surrounded by the dotted lines in the images i401, i402, and i403 are calculated as the contrast values of each of the images.

As described above, the contrast value of the image i401 is the sum of the contrast values corresponding to one focus index image 39b and one focus index image 39c, surrounded by the dotted lines illustrated in Fig. 4. Similarly, the contrast value of the image i402 becomes the sum of the contrast values corresponding to one focus index image 39b and 0.5 of the focus index image 39c. Further, since the focus index images 39b and 39c overlap in the horizontal direction in the image i403, the contrast value corresponds to one focus index image 39b. For example, if the vertical lengths corresponding to one focus index image 39b and to one focus index image 39c are the same, the contrast value of the image i403 becomes 100 × 10 + 100 × 0 = 1000. Further, the contrast value of the image i402 becomes 100 × 10 + 100 × 5 = 1500, and the contrast value of the image i401 becomes 100 × 10 + 100 × 10 = 2000. In other words, the contrast value of the image i403 is the smallest, followed by that of the image i402. The contrast value of the image i401 is thus the greatest. The images i401, i402, and i403 are the images of the area A301 illustrated in Fig. 3, so that the image sizes thereof are the same, and the positions are also the same.

For ease of description, it has been assumed that in the images i401, i402, and i403, the luminance values of the focus index images 39b and 39c are the same, and the luminance values of the portions other than the focus index images 39b and 39c are the same. In the actual fundus observation image, luminance distributions vary depending on an appearance of the focus index images, noise in the portions other than the index images, and the observation image. However, since the focus index images 39b and 39c are projected in high contrast, the luminance difference between the index images and the portions other than the index images becomes dominant in the contrast value.

The method for detecting the position in which a displacement of the focus index images minimizes will be described below with reference to Fig. 5. More specifically, the method uses the method for calculating the contrast values described above with reference to Fig. 4, and the difference in the contrast values due to the positions of the focus index images 39b and 39c.

Referring to Fig. 5, images i501, i502, i503, i504, and i505 indicate the focus index images 39b and 39c in the area A301 illustrated in Fig. 3. Further, the images i501, i502, i503, i504, and i505 indicate the change over time of the focus index images 39b and 39c when the system control unit 18 drives the focus index projection unit 22 over an entire movable range (i.e., an example of a first range). It is not necessary to move the focus index projection unit 22 and the focus lens 12 over the entire movable range. It is only necessary to move the focus index projection unit 22 and the focus lens 12 over the range in which the position where the contrast value becomes a minimum value can be determined.

A graph illustrated in a lower portion of Fig. 5 indicates the transition of the contrast value with respect to the position of the focus index projection unit 22. The graph is a line obtained by connecting the contrast values calculated from each of the images i501, i502, i503, i504, and i505.

As described above with reference to Fig. 4, the contrast value becomes the minimum in the image i503 in which the displacement between the focus index images 39b and 39c is the smallest. In other words, the position of the focus index projection unit 22 corresponding to the image i503 matches the position at which the displacement between the focus index images 39b and 39c becomes the smallest (i.e., the position at which the overlap in the horizontal direction of the first index image and the second index image on the fundus image becomes a minimum). As a result, it is only necessary to detect the position of at least one of the focus lens 12 and the focus index projection unit 22 at which the contrast value obtained from each of the images i501, i502, i503, i504, and i505 becomes the smallest. More specifically, the system control unit 18 drives the focusing unit based on its positional relation with the eye which changes according to focusing states of the first index image and the second index image. The first and second index images are obtained by imaging, using the imaging unit, the return beam from the fundus of the first index image and the second index image. The system control unit 18 thus drives the focusing unit based on the overlap in the horizontal direction of the first index image and the second index image on the fundus image. The position at which the contrast value is approximately smallest may be detected instead of the position at which the contrast value actually becomes smallest. In other words, the position at which the contrast value comes to within a predetermined range around the smallest value may be detected; or less than a predetermined contrast value.

According to the present exemplary embodiment, the focus index images 39b and 39c are arranged as in the images i501, i502, i503, i504, and i505 illustrated in Fig. 5. However, the focus index images may be not viewed as in the images i501, i502, i503, i504, and i505 due to expansion of the movable range of the focus index projection unit 22, and the effect of eyelashes of the subject's eye. In particular, if the displacement between the focus index images 39b and 39c is great, there may be a case where neither of the focus index images 39b and 39c is observable. In such a case, the contrast value calculated by the contrast detection unit 201 becomes smaller than when the focus index images 39b and 39c are aligned in a line in the horizontal direction. Such a state occurs when the position of the focus index projection unit 22 is close to an origin, or becomes more distant from the origin as compared to the position of the focus index projection unit 22 corresponding to the image i505 illustrated in Fig. 5.

As described above, if neither of the focus index images 39b and 39c is observable, the contrast value may be lower than the contrast value obtained from the image i503. The contrast value may be lower even when the displacement amount of the focus index images 39b and 39c is greater than that in the images i501 and i505. However, even in such a case, false detection can be prevented by detecting the position at which the contrast value becomes the smallest as follows. The position at which the contrast value becomes the smallest is detected in a section surrounded by the portions in which the contrast values obtained from the images i501 and i505 become high (i.e., the section surrounded by the portion in which the graph of the contrast value protrudes upwards).

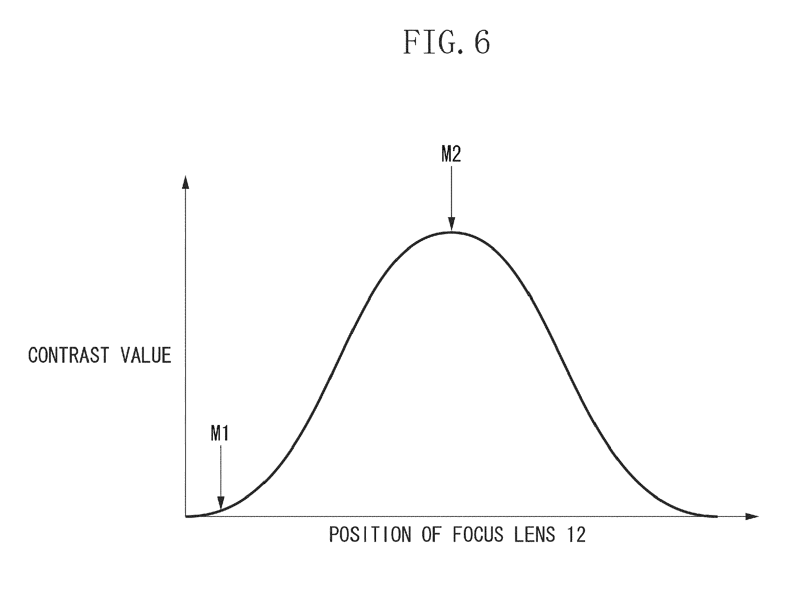

The second focus detection unit 203 will be described in detail below with reference to Fig. 6.

The second focus detection unit 203 detects the focus position using the medium and large blood vessels in the retina in the area A302 illustrated in Fig. 3. Fig. 6 is a graph illustrating the transition of the contrast value with respect to the position of the focus lens 12 moved by the focus lens drive unit 19. When the second focus detection unit 203 is operating, it is not necessary to move the focus index projection unit 22 in conjunction with the movement of the focus lens 12. Further, the contrast value is calculated by the contrast detection unit 201 similarly as described with respect to Fig. 4, so that detailed description will be omitted.

In the method described above with reference to Fig. 4, the difference between the luminance of the portion other than the focus index images 39b and 39c and the luminance of the left lateral side of the focus index image 39c is calculated as the contrast value of the entire image. However, in the method described below with reference to Fig. 6, the difference between the luminance of the portion other than the middle and large blood vessels in the retina and that of both end portions of the middle and large blood vessels will be calculated as the contrast value. For example, the sum of the following differences in the luminance is calculated as the contrast value: The difference between the luminance of a pigment epithelium adjacent to a first end portion of the middle and large blood vessels and the luminance of the first end portion of the middle and large blood vessels, and the difference between the luminance of the pigment epithelium adjacent to a second end portion of the middle and large blood vessels and the luminance of the second end portion of the middle and large blood vessels.

As illustrated in Fig. 6, the contrast value is maximum at a focus position M2, and the contrast value becomes small at a position M1 at which a defocus amount is large. According to the present exemplary embodiment, the focus detection in which the effect of the aberration of the subject' s eye is reduced can be performed using the above-described contrast detection principle. More specifically, the position of the focus lens 12, i.e., the focus position M2, reached by the focus lens drive unit 19 moving the focus lens 12 is the position at which the fundus image displayed on the monitor 15 can be most clearly observed. Further, the position of the focus lens 12, i.e., the focus position M2, reached by the focus lens drive unit 19 moving the focus lens 12 matches the position of the focus lens 12 at which the fundus image displayed on the monitor 15 after being captured can be most clearly displayed.

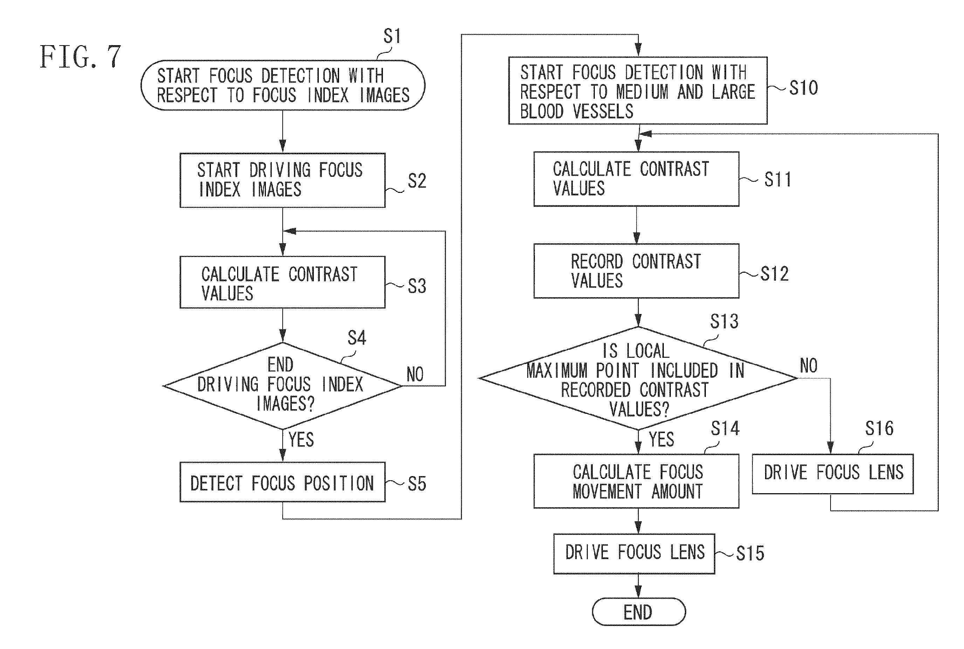

An example of the control method according to the present exemplary embodiment will be described below with reference to the flowchart illustrated in Fig. 7.

The system control unit 18 lights the focus index illumination LED 25, and the focus indices are projected on the fundus. In step S1, the system control unit starts focus detection with respect to the focus index images. In other words, the first focus detection unit 202 starts detecting the focus position. In step S2, the system control unit 18 (i.e., the first focus detection unit 202) drives the focus index projection unit 22 via the focus index drive unit 20.

Step S3 is started approximately at the same time as step S2. In step S3, the contrast detection unit 201 - in synchronization with the first focus detection unit 202 starting the focus detection - detects the contrast value with respect to the area A301 illustrated in Fig. 3. The contrast detection unit 201 performs the detection as described in detail with reference to Figs. 4 and 5. The first focus detection unit 202 may previously determine the area A301 before step S1, or may determine after the process of step S1 has been started. Further, the first focus detection unit 202 records the following in a memory (not illustrated). The first focus detection unit 202 records the contrast value calculated by the contrast detection unit 201, associated with the position of the focus index projection unit 22 or the focus lens 12 when the fundus image of which the contrast value has been calculated is obtained.

In step S4, the system control unit 18 (i.e., the first focus detection unit 202) determines whether the focus index projection unit 22 has reached the end opposite from the starting position. If the system control unit determines that the focus index projection unit 22 has not reached the end (NO in step S4), the process returns to step S3. If the system control unit determines that the focus index projection unit 22 has reached the end (YES in step S4), the first focus detection unit 202 ends driving the focus index projection unit 22, and the process proceeds to step S5.

In step S5, the first focus detection unit 202 detects the position of the focus index projection unit 22 or the focus lens 12 at which the displacement of the focus index images becomes the smallest, based on the contrast value recorded in step S3 and the position of the focus index projection unit 22 or the focus lens 12. The method for detecting the position at which the displacement of the focus index image becomes the smallest is as described above with reference to Figs. 4 and 5. Upon detecting the position at which the displacement of the focus index images becomes the smallest, the system control unit 18 drives the focus index projection unit 22 or the focus lens 12 to the detected position. In other words, the system control unit 18 detects the position at which the overlap in the horizontal direction of the first index image and the second index image in the fundus image becomes the predetermined value or less by driving the focusing unit in the first range. Further, the system control unit 18 drives the focusing unit to the detected position.

As described above, in step S1 to step S5, the position of the focus index projection unit 22 at which the detected displacement of the focus index images 39b and 39c becomes the smallest can be detected. At the same time, the system control unit 18 is capable of controlling the positions of the focus lens 12 at which the focus index projection unit 22 and the image sensor 14 becomes optically conjugate. The focus lens 12 can thus be moved to the position corresponding to the position at which the displacement of the focus index images 39b and 39c becomes the smallest. The processes of step S1 to step S5 correspond to an example of a first driving step. More specifically, in the first driving step, the focus unit, which focuses the return beam from the fundus on the imaging unit, is driven based on the index images obtained by the imaging unit imaging the return beam from the fundus of the indices projected by the projection unit, which projects the indices on the fundus of the subject's eye.

In step S10, the second focus detection unit 203 starts focus detection with respect to the middle and large blood vessels in the fundus based on the position of the focus lens 12, which has been driven in step S5.

The position of the focus lens 12, from which the focus position detection with respect to the middle and large blood vessels in the fundus is started in step S10, will be described below. The moving range of the focus lens 12 may be any range within the range in which the focus position of the middle and large blood vessels in the fundus is contained, based on the position at which the displacement of the focus index images 39b and 39c becomes the smallest. In other words, the second drive unit drives the focusing unit based on the position of the focusing unit driven by the first drive unit. For example, it is assumed that the relation between the position at which the displacement of the focus index images 39b and 39c becomes the smallest and the focus position of the middle and large blood vessels in the fundus is within ±3 diopter. In such a case, the process from step S10 and thereafter are performed within the range of ±3 diopter (i.e., an example of a second range which is narrower than the first range) based on the position of the focus lens 12 which is driven in step S5. According to the present exemplary embodiment, the driving range of the focus lens 12 is set to ±3 diopter in consideration of the effect of the aberration caused by astigmatism. However, it is not limited thereto, and the driving range can be changed to an arbitrary value. For example, if it is previously known that the aberration is large in the subject's eye from past diagnosis information, the driving range may be wider than ±3 diopter. Further, if it is previously known that the aberration is small, the driving range may be narrower than ±3 diopter. In other words, the driving range can be widened as the aberration increases. For example, the information on the aberration may be associated with identification information of the subject, and the driving range of the focus lens 12 may then be controlled according to the subject. In other words, the second range is changeable according to the aberration in the subject's eye, and becomes wider as the aberration increases.

In step S11, the contrast detection unit 201 calculates the contrast with respect to the area A302. In step S12, the second focus detection unit 203 records in the memory (not illustrated) the contrast value calculated in step S11. The second focus detection unit 203 records in the memory (not illustrated) the contrast value calculated by the contrast detection unit 201 in step S11, associated with the position of the focus lens 12 when the fundus image of which the contrast has been calculated is obtained.

In step S13, the second focus detection unit 203 detects whether a local maximum point, i.e., the position M2 illustrated in Fig. 6, is included in the contrast values recorded in step S12.

If the second focus detection unit 203 detects the local maximum point (YES in step S13), the process proceeds to step S14. In step S14, the second focus detection unit 203 calculates a movement amount of the focus lens 12. According to the present exemplary embodiment, the movement amount of the focus lens 12 is a driving amount of the focus lens to the detected position of the local maximum point. For example, if the second focus detection unit 203 records in the memory the contrast value which becomes the local maximum point, the second focus detection unit 203 calculates the movement amount of the focus lens 12 as follows. The difference between the position of the focus lens 12 associated with the contrast value which becomes the local maximum point, and the current position of the focus lens 12 becomes the movement amount of the focus lens 12. If the contrast value which becomes the local maximum point is not recorded in the memory, the following may be performed. The position of the focus lens 12 of when the contrast becomes the maximum may be estimated using the positions of the focus lens 12 associated with the contrast values previous to and subsequent to the maximum contrast value. For example, a middle point of the positions of the focus lens 12 associated with the contrast values previous to and subsequent to the contrast value which becomes the local maximum point is estimated as the position of the focus lens 12 of when the contrast becomes the greatest. As described above, the system control unit 18 detects the position at which the contrast becomes the maximum, and drives the focus lens 12 to the detected position. According to the above-described example, the focus lens 12 is moved to the position at which the contrast value becomes the maximum. However, it is not limited thereto, and the focus lens 12 may be moved to the position at which the contrast value becomes proximate to the maximum. In other words, the focus lens 12 is moved to the position at which the contrast value becomes a predetermined value or greater.

In step S15, the second focus detection unit 203 drives the focus lens 12 via the focus lens drive unit 19 according to the movement amount of the focus lens 12 calculated in step S14. The second focus detection unit 203 thus moves the focus lens 12 to the position at which the contrast value becomes the maximum. In other words, the system control unit 18 drives the focusing unit in the second range, which is narrower than the first range, based on the position of the focusing unit driven by the first drive unit. The system control unit 18 thus detects the position at which the contrast of the fundus image becomes a predetermined value or greater. Further, the system control unit 18 moves the focusing unit to the detected position. According to the present exemplary embodiment, the processes of step S10 to step S15 correspond to an example of the second driving step. More specifically, the focusing unit is driven in the second driving step after the focusing unit has been driven in the first driving step. In the second driving step, the focusing unit is driven based on the contrast of the fundus image obtained by imaging, using the imaging unit, the return beam from the fundus illuminated by the illumination unit.

If the second focus detection unit 203 does not detect the local maximum point (NO in step S13), the process proceeds to step S16. In step S16, only in the case where the focus lens 12 has been driven, the processes of step S11 to step S13 are repeated after the focus lens 12 has been started to be driven by a predetermined amount. The contrast detection of the medium and large blood vessels in the fundus is then repeated.

After driving the focus lens 12 by a predetermined amount in step S16, the second focus detection unit 203 again performs the process of step S13, i.e., detects whether the local maximum point, which is the position M2 illustrated in Fig. 6, is included in the contrast values recorded in step S12.

If the second focus detection unit 203 again does not detect the local maximum point, the process proceeds to step S16. In step S16, only in the case where the focus lens 12 has been driven, the processes of step S11 to step S13 are repeated after the focus lens 12 has been started to be driven by a predetermined amount. The processes of step S16, and step S11 to step S13 are then repeated until the local maximum point is detected.

If the local maximum point is detected in step S13, the processes of step S14 and S15 are performed similarly as described above. As a result, the focus lens 12 is moved to the position at which the contrast becomes the maximum value.

By moving the focus lens 12 as described above, focus adjustment can be performed in accordance with the aberration even when there is individual variation in the aberration of the subject's eye, such as spherical aberration and astigmatism.

As described above, according to the present exemplary embodiment, the position at which the displacement between the focus index images detected becomes the smallest is detected in step S1 to step S5 illustrated in Fig. 7. In step S10 to step S15, the focus position at which the contrast of the medium and large blood vessels in the fundus becomes the maximum is detected.

The system control unit 18 may perform automatic imaging of the subject's eye after performing auto-focusing by detecting the contrast of the focus index images.

Such an operation is particularly effective in a fundus camera which performs observation using infrared light (e.g., a non-mydriatic fundus camera). Since the contrast of the medium and large blood vessels in the fundus is low with respect to the infrared light, there is hardly any difference in the contrast values at the focus lens positions. It is thus difficult to detect the position M2 of the local maximum point. As a result, it becomes necessary to increase the contrast of the observation image as much as possible by driving the focus lens at low speed or repeating stopping and driving. As a result, if the second focus detection unit 203 performs focus detection with respect to the entire driving range of the focus lens, time becomes necessary to perform focusing.

However, if the focus indices in which the contrast is higher than that of the medium and large blood vessels in the fundus are used, it is easy to detect the difference in the contrast values even when the focus lens is driven at high speed. By using such a result, according to the present exemplary embodiment, the first focus detection unit 202 performs focus detection so that the range of the focus detection to be performed by the second focus detection unit 203 becomes limited. The time required for focusing can thus be greatly improved.

According to the present exemplary embodiment, the first focus detection unit using the focus indices allows high-speed focus detection to be performed. The effects of involuntary eye movement and blinking of the subject's eye can thus be greatly reduced. Further, the second focus detection unit detects the focus position with respect to the fundus of the subject's eye. The effect of the aberration of the optical system of the subject's eye such as the astigmatism of the subject's eye can be reduced. In other words, according to the present exemplary embodiment, the effect of the aberration of an imaging target can be improved, and high-speed focus detection can be realized.

Further, according to the present exemplary embodiment, the ophthalmologic apparatus can perform high-speed focus detection with high accuracy. Failure in imaging due to an incorrect focus position is thus prevented, and the time required for imaging the subject's eye can be reduced, so that a load on the subject's eye can be reduced. Further, the apparatus is user-friendly for the operator operating the ophthalmologic apparatus.

According to the first exemplary embodiment, the first focus detection unit 202 performs focus detection using the contrast values of the image, similarly to the second focus detection unit 203. In other words, the first focus detection unit 202 and the second focus detection unit 203 can perform focus detection by detecting the contrast values of the image. Further, the contrast detection positions of the first focus detection unit 202 and the second focus detection unit 203 on the image can be changed, and the respective focus positions can be detected from the contrast value. The focus detection method is thus surprisingly simple.

According to a second exemplary embodiment, the first focus detection unit 202 performs focus detection from the positional relation of the focus index images as will be described below.

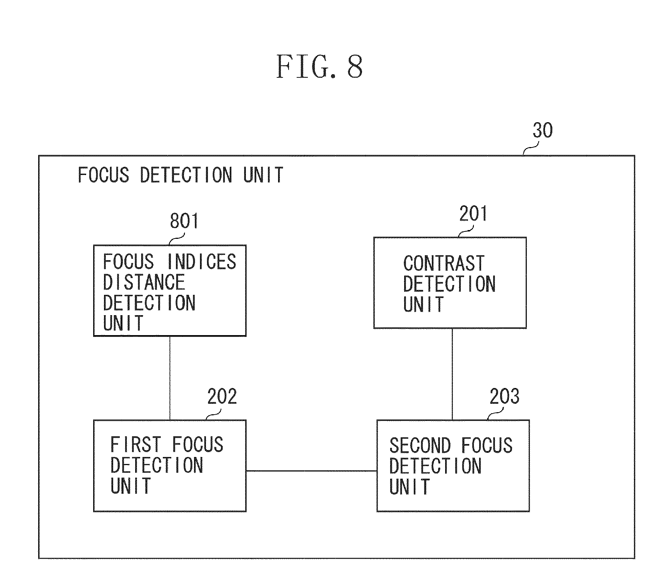

The differences from the first focus exemplary embodiment will be described below with reference to Fig. 8. The elements which are assigned the same reference numbers as the first exemplary embodiment are similar components, so that detailed description will be omitted.

Referring to Fig. 8, according to the second exemplary embodiment, the focus detection unit 30 includes the contrast detection unit 201, the first focus detection unit 202, the second focus detection unit 203, and a focus indices distance detection unit 801. The focus indices distance detection unit 801 is connected to the image sensor 14, to be used for the observation image, and the first focus detection unit 202. It is not necessary for the focus indices distance detection unit 801 to be connected to the image sensor 14, and it may be connected to the image processing unit 17. In such a case, the focus indices distance detection unit 801 obtains the output from the image sensor 14 via the image processing unit 17.

The contrast detection unit 201 is connected to the second focus detection unit 203 and not to the first focus detection unit 202, which is different from the first exemplary embodiment. The first focus detection unit 202 and the second focus detection unit 203 are connected for synchronously starting focus detection, similarly to the first exemplary embodiment. In other words, according to the second exemplary embodiment, the first focus detection unit 202 performs focus detection using the focus indices distance detection unit 801, and the second focus detection unit 203 performs focus detection using the contrast detection unit 201.

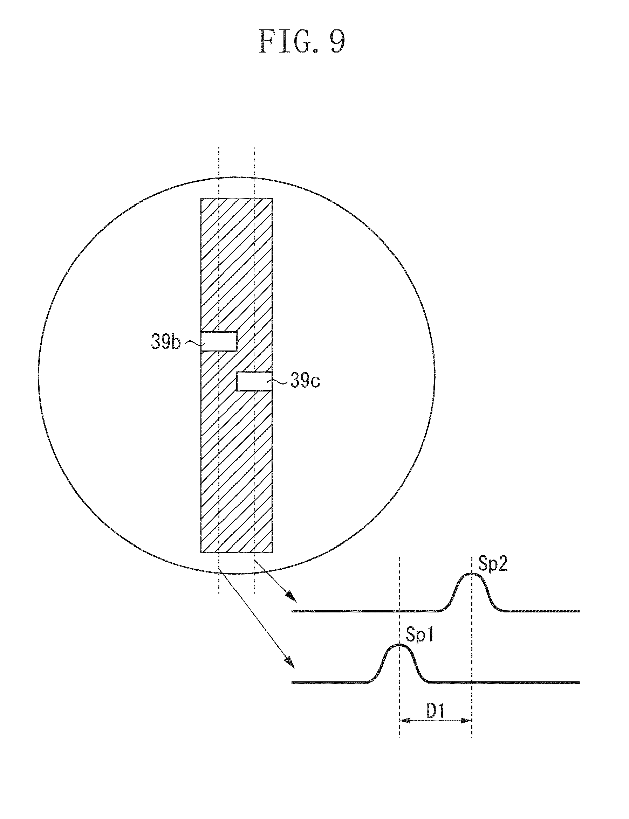

The focus indices distance detection unit 801 calculates the distance between the focus index images 39b and 39c in the area including the focus index images 39b and 39c as illustrated in Fig. 9. More specifically, the focus indices distance detection unit 801 detects the luminance values of a plurality of lines in the vertical direction which respectively runs through the focus index images 39b and 39c in the area including the focus index images 39b and 39c. The focus indices distance detection unit 801 then detects the distance between the focus index images 39b and 39c from the luminance values of each line. More specifically, the focus indices distance detection unit 801 detects a peak position Sp1 of the luminance value of the line running through the focus index image 39b and a peak position Sp2 of the luminance value of the line running through the focus index image 39c.

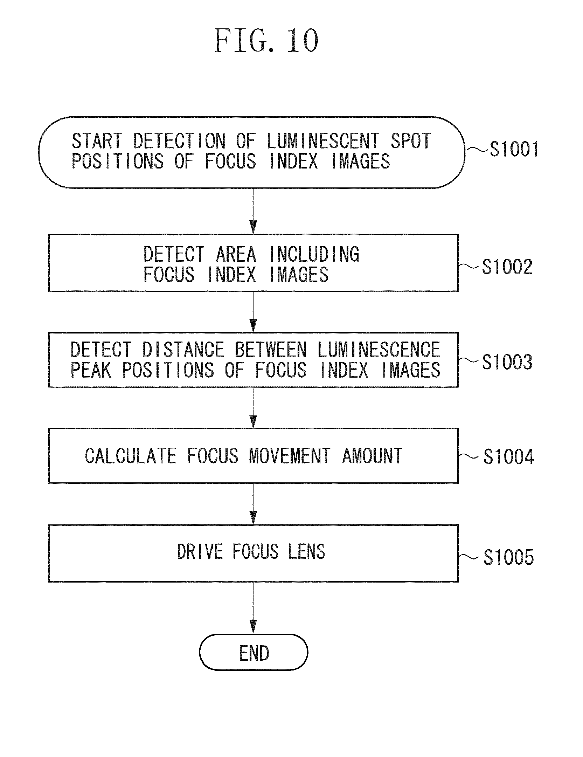

An example of the operation of the ophthalmologic apparatus according to the present exemplary embodiment will be described below with reference to the flowchart illustrated in Fig. 10. The focus index projection unit 22 projects the focus indices on the fundus. In step S1001, the focus indices distance detection unit 801 starts detection of the position of luminance points of the focus index images. In such a case, the focus index images are as illustrated in Fig. 9.

In step S1002, the focus indices distance detection unit 801 detects an area illustrated as a hatched-line area that includes the focus index image 39b toward the left side thereof and the focus index image 39c toward the right edge thereof. Since the positions of the focus index images 39b and 39c may be previously obtained, the area may be detected before performing step S1002. The positions of the focus index images 39b and 39c is almost determined by a design of an optical system illustrated in Fig. 1. Therefore, the hatched-line area can be detected by the focus indices distance detection unit 801.

In step S1003, the focus indices distance detection unit 801 scans the area illustrated in Fig. 9 in the vertical direction. As a result, the focus indices distance detection unit 801 detects the peak position Sp1 of the luminance of the focus index image 39b and the peak position Sp2 of the luminance of the focus index image 39c detected in step S1002. The focus indices distance detection unit 801 then calculates a distance D1 from the positional relation between the two peak positions.

As described above, the focus indices distance detection unit 801 calculates the distance D1 from the positional relation between the focus index images 39b and 39c calculated in the processes of step S1001 to step S1003. The first focus detection unit 202 can then detect the focus position based on the distance D1.

In step S1004, the first focus detection unit 202 calculates the focus movement amount corresponding to the distance D1 calculated in step S1003.

In step S1005, the first focus detection unit 202 drives, via the focus lens drive unit 19, the focus lens 12 according to the driving amount calculated in step S1004.

The processes following step S1005 are similar to those of step S10 to step S16 illustrated in Fig. 7 according to the first exemplary embodiment, so that detailed description will be omitted.

As described above, according to the present exemplary embodiment, the first focus detection unit 202 performs focus detection so that the range of the focus detection to be performed by the second focus detection unit 203 can be limited. As a result, the effect of the aberration can be reduced, and the time necessary for focusing can be shortened, similarly to the first exemplary embodiment.

The system control unit 18 may perform automatic imaging of the subject's eye after completing auto-focusing using the contrast detection of the focus index image.

Techniques of the disclosure are not limited to the above-described exemplary embodiments, and various modifications and alterations are possible without departing from the scope of the invention.

For example, according to the above-described exemplary embodiments, the fundus camera is described as an example. However, the present invention may be applied to ophthalmologic apparatuses such as a slit lamp and optical coherence tomography (OCT) in which the fundus is observed and imaged. In such a case, a similar advantageous effect can be achieved as in the above-described exemplary embodiments.

Further, according to the above-described exemplary embodiments, the focus index images 39b and 39c vertically move according to the focus state. However, it is not limited thereto. For example, the configuration of the focus index projection unit 22 may be changed so that the focus index images 39b and 39c horizontally move according to the focus state.

Embodiments of the present invention can also be realized by a computer of a system or apparatus that reads out and executes computer executable instructions recorded on a storage medium (e.g., non-transitory computer-readable storage medium) to perform the functions of one or more of the above-described embodiment (s) of the present invention, and by a method performed by the computer of the system or apparatus by, for example, reading out and executing the computer executable instructions from the storage medium to perform the functions of one or more of the above-described embodiment (s). The computer may comprise one or more of a central processing unit (CPU), micro processing unit (MPU), or other circuitry, and may include a network of separate computers or separate computer processors. The computer executable instructions may be provided to the computer, for example, from a network or the storage medium. The storage medium may include, for example, one or more of a hard disk, a random-access memory (RAM), a read only memory (ROM), a storage of distributed computing systems, an optical disk (such as a compact disc (CD), digital versatile disc (DVD), or Blu-ray Disc (BD)™), a flash memory device, a memory card, and the like.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

## Claims

1. An ophthalmologic apparatus comprising:
a focusing means for focusing, on an imaging means, a return beam from a fundus of a subject's eye;
a first drive means for driving the focusing means based on an index image obtained by imaging, using the imaging means, a return beam from the fundus resulting from an index projected on the fundus by a projection means; and
a second drive means for driving, after the first drive means has driven the focusing means, the focusing means based on a contrast of a fundus image obtained by imaging, using the imaging means, a return beam from the fundus, the fundus having been illuminated by an illumination means.

2. The ophthalmologic apparatus according to claim 1, wherein the second drive means is operable to drive the focusing means based on a position of the focusing means having been driven by the first drive means.

3. The ophthalmologic apparatus according to claim 1 or 2, wherein the projection means is operable to project, as the index, a first index and a second index on the fundus, and
wherein the first drive means is operable to drive the focusing means based on a positional relation which changes according to focusing states of a first index image and a second index image obtained by imaging, using the imaging means, return beams from the fundus resulting from the first index and the second index.

4. The ophthalmologic apparatus according to claim 3, wherein the first drive means is operable to drive the focusing means based on an overlap in a predetermined direction of the first index image and the second index image on the fundus image.

5. The ophthalmologic apparatus according to claim 4,
wherein the predetermined direction is a horizontal direction,
wherein the first drive means is operable to drive the focusing means within a first range, then to detect a position at which an overlap in the horizontal direction of the first index image and the second index image on the fundus image becomes a predetermined value or less, and to drive the focusing means to the detected position, and
wherein the second drive means is operable to drive, based on a position of the focusing means having been driven by the first drive means, the focusing means within a second range, which is narrower than the first range, then to detect a position at which a contrast of the fundus image becomes a predetermined value or greater, and to drive the focusing means to the detected position.

6. The ophthalmologic apparatus according to claim 5,
wherein the first drive means is operable to detect a position at which an overlap in the horizontal direction of the first index image and the second index image on the fundus image becomes smallest, and
wherein the second drive means is operable to detect a position at which the contrast becomes maximum.

7. The ophthalmologic apparatus according to claim 5 or 6, wherein the second range is changeable according to an aberration in the subject's eye.

8. The ophthalmologic apparatus according to claim 7, wherein the second range becomes wider as the aberration increases.

9. The ophthalmologic apparatus according to any preceding claim, wherein the second drive means is operable to drive the focusing means based on a contrast of a characteristic region included in the fundus image.

10. The ophthalmologic apparatus according to claim 9, wherein the characteristic region is a blood vessel.

11. A control method comprising:
driving, based on an index image obtained by imaging, using an imaging means, a return beam from a fundus of a subject's eye resulting from an index projected on the fundus, a focusing means for focusing, on the imaging means, the return beam from the fundus of the subject's eye; and
after the focusing means has been driven, driving the focusing means based on a contrast of a fundus image obtained by imaging, using the imaging means, a return beam from the fundus, the fundus having been illuminated.

12. A program that causes a computer to perform the control method according to claim 11.
